(19) Europäisches Patentamt
European Patent Office
Office européen des brevets

(11) Veröffentlichungsnummer : **0 108 995**
**B1**

(12) # EUROPÄISCHE PATENTSCHRIFT

(45) Veröffentlichungstag der Patentschrift : **28.05.86**

(21) Anmeldenummer : **83110888.1**

(22) Anmeldetag : **02.11.83**

(51) Int. Cl.⁴ : **C 07 D249/08**, C 07 D233/60, C 07 D405/06, A 01 N 43/653, A 01 N 43/50

(54) **Hydroxyalkinyl-azolyl-Derivate.**

(30) Priorität : **15.11.82 DE 3242222**

(43) Veröffentlichungstag der Anmeldung : **23.05.84 Patentblatt 84/21**

(45) Bekanntmachung des Hinweises auf die Patenterteilung : **28.05.86 Patentblatt 86/22**

(84) Benannte Vertragsstaaten : **AT BE CH DE FR GB IT LI NL SE**

(56) Entgegenhaltungen :
**EP-A- 0 021 327**
**EP-A- 0 021 345**
**EP-A- 0 043 419**
**EP-A- 0 057 864**
**DE-A- 3 025 308**
**DE-A- 3 048 267**
**DE-A- 3 106 076**
**DE-A- 3 124 580**
**DE-A- 3 141 819**
**US-A- 4 321 272**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Lantzsch, Reinhard, Dr.**
**Heymannstrasse 32**
**D-5090 Leverkusen 1 (DE)**
Erfinder : **Ditgens, Klaus, Dr.**
**Obere Rutenbeck 95**
**D-5600 Wuppertal 12 (DE)**
Erfinder : **Büchel, Karl Heinz, Prof. Dr.**
**Dabringhausener Strasse 42**
**D-5093 Burscheid (DE)**
Erfinder : **Reinecke, Paul, Dr.**
**Lessingstrasse 11**
**D-5090 Leverkusen 3 (DE)**
Erfinder : **Brandes, Wilhelm, Dr.**
**Eichendorffstrasse 3**
**D-5653 Leichlingen (DE)**

**EP 0 108 995 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Hydroxyalkinyl-azolyl-Derivate, ein Verfahren zu ihrer Herstellung sowie ihre Verwendung als Fungizide.

Es ist bereits bekannt geworden, daß bestimmte 3,3-Dimethyl-1-(1,2,4-triazol-1-yl)-2-butanole, wie beispielsweise 2-(4-Biphenylyloxymethyl)-3,3-dimethyl-1-(1,2,4-triazol-1-yl)-2-butanol bzw. 2-(4-Chlorphenyl-acetylenyl)-3,3-dimethyl-1-(1,2,4-triazol-1-yl)-2-butanol, gute fungizide Eigenschaften aufweisen (vgl. DE-A 3 018 866 bzw. EP-A 0 052 424). Die Wirkung dieser Verbindungen ist jedoch, insbesondere bei niedrigen Aufwandmengen und -konzentrationen, nicht immer ganz befriedigend.

Aus der EP-A 0 021 327 und der EP-A 0 021 345 sind fungizid wirksame Ethyl-azolyl-Derivate bekannt, die in 2-Stellung zum Azolyl-Rest eine Alkinyl-Gruppe enthalten können. Verbindungen dieses Typs, in denen der Azolyl-Rest über eine $CH_2$-Gruppe mit dem verbleibenden Molekülteil verbunden ist, werden jedoch nicht offenbart.

Ferner werden in der DE-A 31 24 580 2-Hydroxyethyl-azolyl-Derivate mit fungiziden Eigenschaften beschrieben. Es werden aber keine Stoffe erwähnt, in denen der Azolyl-Rest über eine unsubstituierte Methylgruppe mit dem Carbinol-Kohlenstoffatom verknüpft ist.

Es wurden neue Hydroxyalkinyl-azolyl-Derivate der allgemeinen Formel (I)

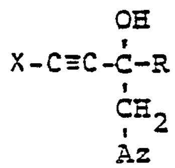

(I)

in welcher

Az für 1,2,4-Triazol-1-yl oder Imidazol-1-yl steht,

X für geradkettiges oder verzweigtes Alkyl mit 1 bis 4 Kohlenstoffatomen oder für gegebenenfalls einfach bis fünffach, gleich oder verschieden durch Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen, den Aldoximrest, den Ketoximrest und deren Ether-Derivate, sowie jeweils gegebenenfalls durch Halogen und Alkyl mit 1 bis 2 Kohlenstoffatomen substituiertes Phenyl, Phenoxy, Benzyl und Benzyloxy, substituiertes Phenyl steht,

R für die Gruppierungen

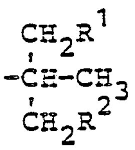 , 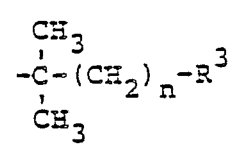 und 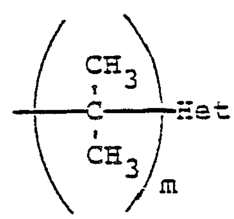

wobei

$R^1$ für Wasserstoff, Fluor, Chlor oder Brom steht,

$R^2$ für Fluor, Chlor oder Brom steht,

$R^3$ für Alkylthio mit 1 bis 4 Kohlenstoffatomen, für Halogenalkoxy und Halogenalkylthio mit jeweils 1 bis 2 Kohlenstoffatomen und 1 bis 5 gleichen oder verschiedenen Halogenatomen, für Alkenyl mit 2 bis 6 Kohlenstoffatomen, für Alkoxycarbonyl mit 1 bis 4 Kohlenstoffatomen im Alkylteil, für Cyano sowie für jeweils gegebenenfalls einfach bis fünffach, gleich oder verschieden substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil steht, wobei jeweils als Phenylsubstituenten genannt seien : Halogen, Alkyl mit 1 bis 4 Kohlenstoffatomen ; Alkoxy und Alkylthio mit jeweils 1 bis 2 Kohlenstoffatomen ; Halogenalkyl, Halogenalkoxy und Halogenalkylthio mit jeweils 1 bis 2 Kohlenstoffatomen und 1 bis 5 gleichen oder verschiedenen Halogenatomen, Cyclohexyl, Dialkylamino mit 1 bis 4 Kohlenstoffatomen in jedem Alkylteil, Nitro, Cyano, Alkoxycarbonyl mit 1 bis 4 Kohlenstoffatomen im Alkylteil, sowie gegebenenfalls durch Halogen substituiertes Phenyl ;

n für die Zahlen 0, 1 oder 2 steht,

Het für jeweils gegebenenfalls einfach bis vierfach, gleich oder verschieden substituiertes Dioxolan-2-yl oder 1,3-Dioxanyl steht, wobei als Substituenten genannt seien : Alkyl mit 1 bis 4 Kohlenstoffatomen sowie jeweils gegebenenfalls einfach bis fünffach, gleich oder verschieden substituiertes Phenyl und Phenoxyalkyl mit 1 bis 4 Kohlenstoffatomen im Alkylteil, wobei als Phenylsubstituenten genannt seien : Halogen, Alkyl mit 1 bis 4 Kohlenstoffatomen, Alkoxy und Alkylthio mit jeweils 1 bis 2 Kohlenstoffatomen sowie Halogenalkyl, Halogenalkoxy und Halogenalkylthio mit jeweils 1 bis 2 Kohlenstoffatomen und 1 bis 5 gleichen oder verschiedenen Halogenatomen, und

m für die Zahlen 0 oder 1 steht,

sowie deren Säureadditions-Salze und Metallsalz-Komplexe, gefunden.

Die Verbindungen der Formel (I) besitzen ein asymmetrisches Kohlenstoffatom und können deshalb in den beiden optischen Isomerenformen anfallen.

Weiterhin wurde gefunden, daß man die neuen Hydroxyalkinyl-azolyl-Derivate der allgemeinen Formel (I) sowie deren Säureadditions-Salze und Metallsalz-Komplexe erhält, wenn man Azolyl-ketone der allgemeinen Formel (II)

$$Az—CH_2—CO—R \qquad (II)$$

in welcher Az und R die oben angegebene Bedeutung haben, mit Acetylen-Derivaten der allgemeinen Formel (III)

$$X—C \equiv C—H \qquad (III)$$

in welcher X die oben angegebene Bedeutung hat, in Gegenwart einer Base und eines Verdünnungsmittels sowie gegebenenfalls in Gegenwart eines Phasentransferkatalysators umsetzt und an die so erhaltenen Verbindungen der Formel (I) gegebenenfalls anschließend eine Säure oder ein Metallsalz addiert.

Außerdem wurde gefunden, daß die Hydroxyalkinyl-azolyl-Derivate der allgemeinen Formel (I) starke fungizide Eigenschaften aufweisen.

Überraschenderweise zeigen die erfindungsgemäßen Hydroxy-alkinyl-azolyl-Derivate der Formel (I) bessere fungizide Wirkungen, als die obengenannten, aus dem Stand der Technik bekannten 3,3-Dimethyl-1-(1,2,4-triazol-1-yl)-2-butanole, welche konstitutionell und wirkungsmäßig naheliegende Verbindungen sind. Die erfindungsgemäßen Wirkstoffe stellen somit eine Bereicherung der Technik dar.

Die erfindungsgemäßen Hydroxyalkinyl-azolyl-Derivate sind durch die Formel (I) allgemein definiert. Sie unterscheiden sich von denjenigen Verbindungen, die aus der EP-A 0 021 327 und der EP-A 0 021 345 bekannt sind bzw. in der vorgängigen aber nicht vorveröffentlichten DE-A 31 24 580 beschrieben werden, in erster Linie dadurch, daß der Azolyl-Rest über eine unsubstituierte Methylen-Gruppe mit dem verbleibenden Molekülteil verbunden ist.

Bevorzugt sind diejenigen Verbindungen der Formel (I), in denen

Az für 1,2,4-Triazol-1-yl oder Imidazol-1-yl steht ;

X für Methyl, Ethyl, tert.-Butyl sowie für gegebenenfalls einfach bis dreifach, gleich oder verschieden substituiertes Phenyl steht, wobei als Substituenten genannt seien : Fluor, Chlor, Brom, Methyl, tert.-Butyl, Methoxy, Methylthio, Trifluormethyl, Trifluormethoxy, Trifluormethylthio, Hydroximinomethyl, 1-Hydroximinoethyl, Methoximinomethyl, 1-Methoximinoethyl sowie jeweils gegebenenfalls durch Fluor, Chlor und/oder Methyl substituiertes Phenyl, Phenoxy, Benzyl oder Benzyloxy ; und

R für die Gruppierungen

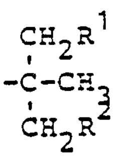 , 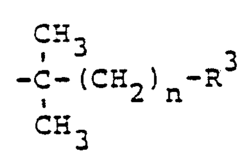 und 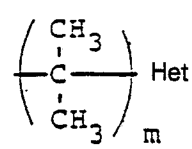

steht, wobei

$R^1$ für Wasserstoff, Fluor oder Chlor steht ;

$R^2$ für Fluor oder Chlor steht ;

$R^3$ für Methylthio, Ethylthio, Trifluormethoxy, Trifluormethylthio, Vinyl, Methoxycarbonyl, Ethoxycarbonyl, Cyano sowie für jeweils gegebenenfalls einfach oder zweifach, gleich oder verschieden substituiertes Phenyl, Phenoxy, Phenylthio, Phenylmethoxy oder Phenylmethylthio steht, wobei jeweils als Phenylsubstituenten genannt seien : Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl ;

n für die Zahlen 0, 1 oder 2 steht ;

Het für jeweils gegebenenfalls einfach bis dreifach, gleich oder verschieden substituiertes Dioxolan-2-yl, 1,3-Dioxan-5-yl oder 1,3-Dioxan-2-yl steht, wobei als Substituenten genannt seien : Methyl, Ethyl, n-Propyl, Isopropyl sowie jeweils gegebenenfalls einfach bis dreifach, gleich oder verschieden durch Fluor, Chlor, Methyl, Trifluormethyl oder Trifluormethoxy substituiertes Phenyl und Phenoxymethyl und

m für die Zahlen 0 oder 1 steht.

Bevorzugte erfindungsgemäße Verbindungen sich auch Additionsprodukte aus Säuren und denjenigen Hydroxyalkinylazolyl-Derivaten der Formel (I), in denen die Substituenten Az, R und X die Bedeutungen haben, die bereits als bevorzugt für diese Substituenten genannt wurden.

Zu den Säuren die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z. B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Hydroxyalkinyl-azolyl-Derivaten der Formel (I), in denen die Substituenten Az, R und X die Bedeutungen haben, die bereits als bevorzugt für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte Säuren sind in diesem Zusammenhang die Halogenwasserstoffsäuren, wie z. B. die Chlorwasserstoffsäure und die Bromwasserstoffsäure, ferner Phosphorsäure, Salpetersäure und Schwefelsäure.

Verwendet man beispielsweise 3,3-Dimethyl-4-fluor-1-(1,2,4-triazol-1-yl)-2-butanon und 4-Chlorphenyl-acetylen als Ausgangsstoffe, so kann der Ablauf des erfindungsgemäßen Verfahrens durch das folgende Formelschema wiedergegeben werden :

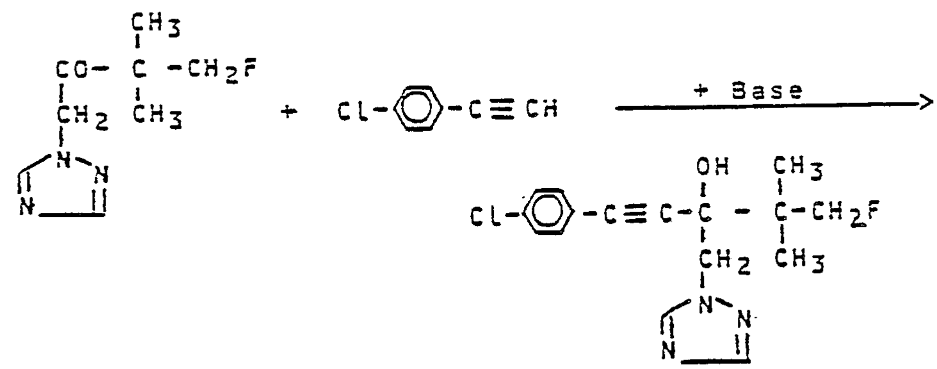


Die bei der Durchführung des erfindungsgemäßen Verfahrens als Ausgangsstoffe zu verwendenden Azolyl-Ketone sind durch die Formel (II) allgemein definiert.

Die Azolyl-ketone der allgemeinen Formel (II) sind bekannt (vergleiche beispielsweise DE-A 2 820 361, DE-A 3 048 266 und EP-A 0 043 923) bzw. sind Gegenstand eigener älterer Patentanmeldungen (vg. DE-A 32 22 220 und DE-A 32 24 129) bzw. lassen sie sich nach im Prinzip bekannten Verfahren herstellen bzw. lassen sie sich nach im Prinzip bekannten Verfahren herstellen.

Die außerdem für das erfindungsgemäße Verfahren als Ausgangsstoffe zu verwendenden Acetylen-Derivate sind durch die Formel (III) allgemein definiert.

Die Acetylen-Derivate der Formel (III) sind allgemein bekannte Verbindungen der organischen Chemie.

Als Verdünnungsmittel kommen für das erfindungsgemäße Verfahren inerte organische Lösungsmittel in Frage.

Hierzu gehören vorzugsweise Ether, wie Tetrahydrofuran, Dioxan, Ethylenglykoldiethylether, Diethylenglykoldimethylether oder Diethylether ; aromatische Kohlenwasserstoffe, wie Benzol, Chlorbenzol, o-Dichlorbenzol oder Toluol ; aliphatische Kohlenwasserstoffe, wie Hexan, Cyclohexan oder Methylenchlorid ; Säureamide, wie Dimethylformamid oder Hexamethylphosphorsäure-triamid ; Sulfoxide, wie Dimethylsulfoxid ; Nitrile, wie Acetonitril ; und Acetale, wie Butyraldehyddibutylacetal, Acetaldehyddibutylacetal oder Methylethyldioxolan.

Die erfindungsgemäße Umsetzung wird in Gegenwart einer starken Base durchgeführt. Hierzu gehören vorzugsweise Alkalimetallamide, -hydride und -hydroxide, wie beispielsweise Natriumamid, -hydroxid oder -hydrid und Kaliumamid, -hydroxid oder -hydrid ; ferner Alkalialkoholate, wie Kalium-tert.-butylat ; Grignard-Verbindungen, wie Ethylmagnesiumbromid ; oder andere metallorganische Verbindungen, wie Butyllithium.

Die erfindungsgemäße Umsetzung wird gegebenenfalls in Gegenwart eines Phasentransferkatalysators durchgeführt, wie vorzugsweise einer Ammonium- oder Phosphoniumverbindung, beispielsweise seien Tetrabutylammoniumbromid oder -chlorid und Triethylbenzyl-ammoniumchlorid genannt.

Die Reaktionstemperaturen können bei der Durchführung des erfindungsgemäßen Verfahrens in einem größeren Bereich variiert werden. Im allgemeinen arbeitet man zwischen — 80 und + 80 °C, vorzugsweise bei — 10 bis + 40 °C. Bei der Verwendung eines Phasentransferkatalysators arbeitet man zwischen 40 und 150 °C, vorzugsweise von 70 bis 120 °C.

Bei der Durchführung des erfindungsgemäßen Verfahrens setzt man vorzugsweise äquimolare Mengen der Ausgangsprodukte ein. Isolierung der Endprodukte erfolgt in üblicher Weise.

In einer besonderen Ausführungsform wird die erfindungsgemäße Umsetzung in einem Zweiphasensystem, wie beispielsweise wäßriger Natron- oder Kalilauge/Toluol oder Methylenchlorid durchgeführt, gegebenenfalls unter Zusatz von 0,1-1 Mol eines Phasentransferkatalysators.

Zur Herstellung von Säureadditions-Salzen der Verbindungen der allgemeinen Formel (I) kommen

vorzugsweise diejenigen Säuren in Frage, die bereits im Zusammenhang mit der Beschreibung der erfindungsgemäßen Säureadditions-Salze als bevorzugte Säuren genannt wurden.

Die Säureadditionssalze der Verbindungen der Formel (I) können in einfacher Weise nach üblichen Salzbildungsmethoden, z. B. durch Lösen einer Verbindung der allgemeinen Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z. B. durch Lösen des Metallsalzes in Alkohol, z. B. Ethanol und Hinzufügen zu Verbindungen der allgemeinen Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie gegen echten Mehltau an Gerste (Erysiphe graminis) oder gegen Braunrost an Weizen (Puccinia recondita) ; von Uromyces-Arten, wie gegen den Erreger des Bohnenrostes (Uromyces appendiculatus) ; sowie von Reiskrankheiten, wie Pyricularia orizae und Pellicularia sasakii, eingesetzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsions-Konzentrate, Saatgutpuder, Wirkstoff-imprägnierte Naturstoffe und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln ; als Emulgier- und/oder schaumerzeugende Mittel kommen in Frage : z. B. nicht-inonogene und anionische Emulgatoren, wie Polyoxyethylen-Fettsäureester, Polyoxyethylen-Fettalkohol-ether, z. B. Alkylaryl-polyglykol-ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden,

Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je kg Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

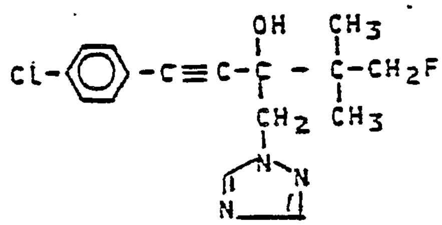

Zu 13,65 g (0,1 Mol) 4-Chlorphenyl-acetylen in 50 ml absolutem Tetrahydrofuran tropft man bei 0 °C unter Stickstoff 62 ml Butyllithium (15 %ig in Hexan, 0,1 Mol). Man läßt 15 Minuten bei 0 °C nachrühren und versetzt dann tropfenweise mit 18,5 g (0,1 Mol) 3,3-Dimethyl-4-fluor-1-(1,2,4-triazol-1-yl)-2-butanon in 30 ml absolutem Tetrahydrofuran. Danach läßt man das Reaktionsgemisch auf Raumtemperatur erwärmen und rührt 14 Stunden nach. Man engt durch teilweises Abdestillieren des Lösungsmittels ein, nimmt den Rückstand in Ether auf, wäscht mit Wasser neutral, trocknet über Natriumsulfat und engt ein. Der Rückstand wird aus Diisopropylether umkristallisiert. Man erhält 19,9 g (61,9 % der Theorie) 1-(4-Chlorphenyl)-4,4-dimethyl-5-fluor-3-(1,2,4-triazol-1-yl-methyl)-1-pentin-3-ol vom Schmelzpunkt 120 °C.

In analoger Weise und entsprechend den angegebenen Verfahrensbedingungen werden die nachfolgenden Verbindungen der allgemeinen Formel (I)

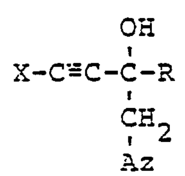 (I)

erhalten

| Bsp. Nr. | R | X | Az | Schmelzpunkt (°C) |
|---|---|---|---|---|
| 2 | $-C(CH_3)_2-CH_2Cl$ | 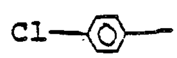 | 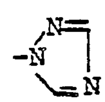 | 129 |
| 3 | $-C(CH_2F)_2-CH_3$ | 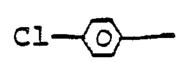 | 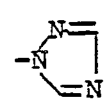 | 128 |
| 4 | 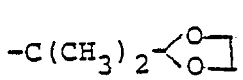 | 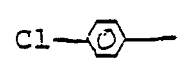 | 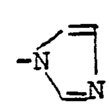 | 143 |

Fortsetzung

| Bsp. Nr. | R | X | Az | Schmelzpunkt (°C) |
|---|---|---|---|---|
| 5 | $-C(CH_3)_2-$ 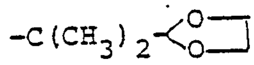 | 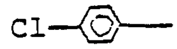 | 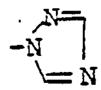 | 86 |
| 6 | $-C(CH_3)_2-$ 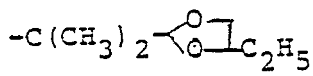 | 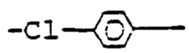 | 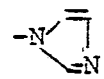 | 103 |
| 7 | $-C(CH_3)_2-$ 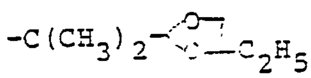 | 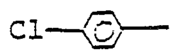 | 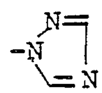 | 97 |
| 8 | $-C(CH_3)_2-$ 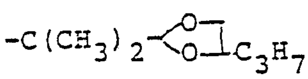 | 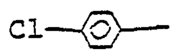 | 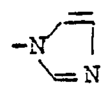 | 81-83 |
| 9 | $-C(CH_3)_2-$ 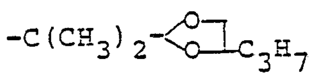 | 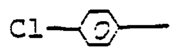 | 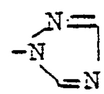 | 93 |
| 10 | $-C(CH_3)_2-$ 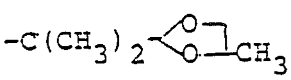 | 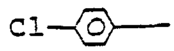 | 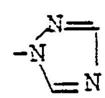 | 96 |
| 11 | $-C(CH_3)_2-$ 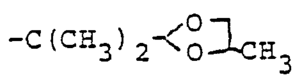 | 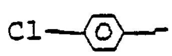 | 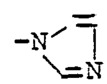 | 101 |
| 12 | $-C(CH_3)_2-CH_2F$ | $(CH_3)_3C-$ | 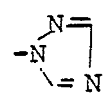 | 101 |

## Verwendungsbeispiele

In den nachfolgenden Verwendungsbeispielen werden die nachstehend angegebenen bekannten Substanzen als Vergleichsverbindungen eingesetzt :

(A)

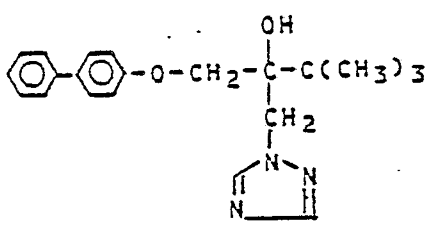

(B)

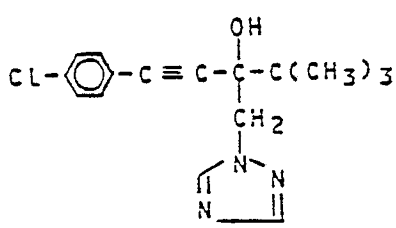

Beispiel A

Erysiphe-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 2, 1 und 3.

Beispiel B

Puccinia-Test (Weizen)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20 °C und 100 % rel. Luftfeuchtigkeit in einer Inkubationskabine.

Die Pflanzen werden in einem Gewächshaus bei einer Temperatur von ca. 20 °C und einer rel. Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 1 und 3.

Beispiel C

Uromyces-Test (Buschbohne)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Uredosporensuspension des Bohnenrosterregers (Uromyces appendiculatus) inokuliert und verbleiben 1 Tag in einer dunklen Feuchtkammer bei 20 bis 22 °C und 100 % rel. Luftfeuchtigkeit.

Die Pflanzen werden dann unter intensiver Belichtung für 9 Tage bei 20 bis 22 °C und einer rel. Luftfeuchtigkeit von 70 bis 80 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 2,1 und 3.

**Patentansprüche**

1. Hydroxyalkinyl-azolyl-Derivate der allgemeinen Formel

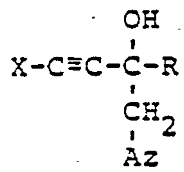 (I)

in welcher

Az für 1,2,4-Triazol-1-yl oder Imidazol-1-yl steht,

X für geradkettiges oder verzweigtes Alkyl mit 1 bis 4 Kohlenstoffatomen oder für gegebenenfalls einfach bis fünffach, gleich oder verschieden durch Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen, den Aldoximrest, den Ketoximrest und deren Ether-Derivate, sowie jeweils gegebenenfalls durch Halogen und Alkyl mit 1 bis 2 Kohlenstoffatomen substituiertes Phenyl, Phenoxy, Benzyl und Benzyloxy, substituiertes Phenyl steht,

R für die Gruppierungen

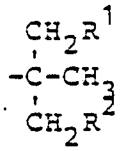 , 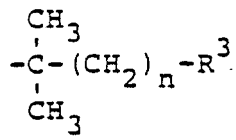 und 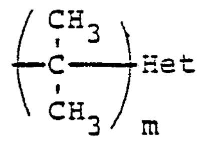

steht, wobei

$R^1$ für Wasserstoff, Fluor, Chlor oder Brom steht,

$R^2$ für Fluor, Chlor oder Brom steht,

$R^3$ für Alkylthio mit 1 bis 4 Kohlenstoffatomen, für Halogenalkoxy und Halogenalkylthio mit jeweils 1 bis 2 Kohlenstoffatomen und 1 bis 5 gleichen oder verschiedenen Halogenatomen, für Alkenyl mit 2 bis 6 Kohlenstoffatomen, für Alkoxycarbonyl mit 1 bis 4 Kohlenstoffatomen im Alkylteil, für Cyano sowie für jeweils gegebenenfalls einfach bis fünffach, gleich oder verschieden substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil steht, wobei jeweils als Phenylsubstituenten genannt seien : Halogen, Alkyl mit 1 bis 4 Kohlenstoffatomen ; Alkoxy und Alkylthio mit jeweils 1 bis 2 Kohlenstoffatomen ; Halogenalkyl, Halogenalkoxy und Halogenalkylthio mit jeweils 1 bis 2 Kohlenstoffatomen und 1 bis 5 gleichen oder verschiedenen Halogenatomen, Cyclohexyl, Dialkylamino mit 1 bis 4 Kohlenstoffatomen in jedem Alkylteil, Nitro, Cyano, Alkoxycarbonyl mit 1 bis 4 Kohlenstoffatomen im Alkylteil, sowie gegebenenfalls durch Halogen substituiertes Phenyl ;

n für die Zahlen 0, 1 oder 2 steht,

Het für jeweils gegebenenfalls einfach bis vierfach, gleich oder verschieden substituiertes Dioxolan-2-yl oder 1,3-Dioxanyl steht, wobei als Substituenten genannt seien : Alkyl mit 1 bis 4 Kohlenstoffatomen sowie jeweils gegebenenfalls einfach bis fünffach, gleich oder verschieden substituiertes Phenyl und Phenoxyalkyl mit 1 bis 4 Kohlenstoffatomen im Alkylteil, wobei als Phenylsubstituenten genannt seien : Halogen, Alkyl mit 1 bis 4 Kohlenstoffatomen, Alkoxy und Alkylthio mit jeweils 1 bis 2 Kohlenstoffatomen sowie Halogenalkyl, Halogenalkoxy und Halogenalkylthio mit jeweils 1 bis 2 Kohlenstoffatomen und 1 bis 5 gleichen oder verschiedenen Halogenatomen, und

m für die Zahlen 0 oder 1 steht,

sowie deren Säureadditions-Salze und Metallsalz-Komplexe.

2. Hydroxyalkinyl-azolyl-Derivate der Formel (I) in Anspruch 1, wobei

Az für 1,2,4-Triazol-1-yl oder Imidazol-1-yl steht,

X für Methyl, Ethyl, tert.-Butyl sowie für gegebenenfalls einfach bis dreifach, gleich oder verschieden substituiertes Phenyl steht, wobei als Substituenten genannt seien : Fluor, Chlor, Brom, Methyl, tert.-Butyl, Methoxy, Methylthio, Trifluormethyl, Trifluormethoxy, Trifluormethylthio, Hydroximinomethyl, 1-Hydroximinoethyl, Methoximinomethyl, 1-Methoximinoethyl sowie jeweils gegebenenfalls durch Fluor, Chlor und Methyl substituiertes Phenyl, Phenoxy, Benzyl oder Benzyloxy,

R für die Gruppierungen

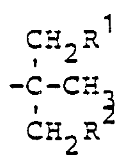 , 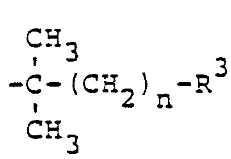 und 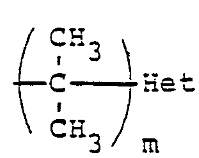

steht, wobei

$R^1$ für Wasserstoff, Fluor oder Chlor steht,

$R^2$ für Fluor oder Chlor steht,

$R^3$ für Methylthio, Ethylthio, Trifluormethoxy, Trifluormethylthio, Vinyl, Methoxycarbonyl, Ethoxycarbonyl, Cyano sowie für jeweils gegebenenfalls einfach bis zweifach, gleich oder verschieden substituiertes Phenyl, Phenoxy, Phenylthio, Phenylmethoxy oder Phenylmethylthio steht, wobei jeweils als Phenylsubstituenten genannt seien : Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl,

n für die Zahlen 0, 1 oder 2 steht,

Het für jeweils gegebenenfalls einfach bis dreifach, gleich oder verschieden substituiertes Dioxolan-2-yl, 1,3-Dioxan-5-yl oder 1,3-Dioxan-2-yl steht, wobei als Substituenten genannt seien : Methyl, Ethyl, n-Propyl, Isopropyl sowie jeweils gegebenenfalls einfach bis dreifach, gleich oder verschieden durch Fluor, Chlor, Methyl, Trifluormethyl oder Trifluormethoxy substituiertes Phenyl und Phenoxymethyl, und

m für die Zahlen 0 oder 1 steht.

3. Verfahren zur Herstellung von Hydroxyalkinyl-azolyl-Derivaten der allgemeinen Formel

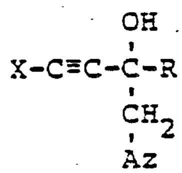

(I)

in welcher

Az für 1,2,4-Triazol-1-yl oder Imidazol-1-yl steht,

X für geradkettiges oder verzweigtes Alkyl mit 1 bis 4 Kohlenstoffatomen oder für gegebenenfalls einfach bis fünffach, gleich oder verschieden durch Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen, den Aldoximrest, den Ketoximrest und deren Ether-Derivate, sowie jeweils gegebenenfalls durch Halogen und Alkyl mit 1 bis 2 Kohlenstoffatomen substituiertes Phenyl, Phenoxy, Benzyl und Benzyloxy, substituiertes Phenyl steht,

R für die Gruppierungen

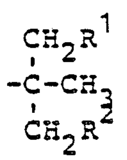

,

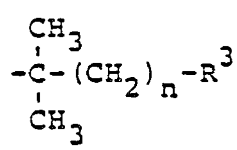

und

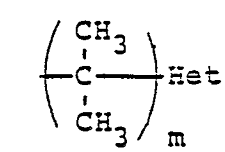

steht, wobei

$R^1$ für Wasserstoff, Fluor, Chlor oder Brom steht,

$R^2$ für Fluor, Chlor oder Brom steht,

$R^3$ für Alkylthio mit 1 bis 4 Kohlenstoffatomen, für Halogenalkoxy und Halogenalkylthio mit jeweils 1 bis 2 Kohlenstoffatomen und 1 bis 5 gleichen oder verschiedenen Halogenatomen, für Alkenyl mit 2 bis 6 Kohlenstoffatomen, für Alkoxycarbonyl mit 1 bis 4 Kohlenstoffatomen im Alkylteil, für Cyano sowie für jeweils gegebenenfalls einfach bis fünffach, gleich oder verschieden substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil steht, wobei jeweils als Phenylsubstituenten genannt seien : Halogen, Alkyl mit 1 bis 4 Kohlenstoffatomen ; Alkoxy und Alkylthio mit jeweils 1 bis 2 Kohlenstoffatomen ; Halogenalkyl, Halogenalkoxy und Halogenalkylthio mit jeweils 1 bis 2 Kohlenstoffatomen und 1 bis 5 gleichen oder verschiedenen Halogenatomen, Cyclohexyl, Dialkylamino mit 1 bis 4 Kohlenstoffatomen in jedem Alkylteil, Nitro, Cyano, Alkoxycarbonyl mit 1 bis 4 Kohlenstoffatomen im Alkylteil, sowie gegebenenfalls durch Halogen substituiertes Phenyl ;

n für die Zahlen 0, 1 oder 2 steht,

Het für jeweils gegebenenfalls einfach bis vierfach, gleich oder verschieden substituiertes Dioxolan-2-yl oder 1,3-Dioxanyl steht, wobei als Substituenten genannt seien : Alkyl mit 1 bis 4 Kohlenstoffatomen sowie jeweils gegebenenfalls einfach bis fünffach, gleich oder verschieden substituiertes Phenyl und Phenoxyalkyl mit 1 bis 4 kohlenstoffatomen im Alkylteil, wobei als Phenylsubstituenten genannt seien : Halogen, Alkyl mit 1 bis 4 Kohlenstoffatomen, Alkoxy und Alkylthio mit jeweils 1 bis 2 Kohlenstoffatomen sowie Halogenalkyl, Halogenalkoxy und Halogenalkylthio mit jeweils 1 bis 2 Kohlenstoffatomen und 1 bis 5 gleichen oder verschiedenen Halogenatomen, und

m für die Zahlen 0 oder 1 steht,

sowie deren Säureadditions-Salze und Metallsalz-Komplexe, dadurch gekennzeichnet, daß man Azolylketone der allgemeinen Formel (II)

$$Az—CH_2—CO—R \quad (II)$$

in welcher Az und R die oben angegebene Bedeutung haben, mit Acetylen-Derivaten der allgemeinen Formel (III)

$$X—C{\equiv}C—H \quad (III)$$

in welcher X die oben angegebene Bedeutung hat,
in Gegenwart einer Base und eines Verdünnungsmittels sowie gegebenenfalls in Gegenwart eines Phasentransferkatalysators umsetzt und gegebenenfalls an die erhaltenen Verbindungen der Formel (I) eine Säure oder ein Metallsalz addiert.

4. Pflanzenschutzmittel, gekennzeichnet durch einen Gehalt an mindestens einem Hydroxyalkinyl-azolyl-Derivat der Formel (I) in Ansprüchen 1 und 3 bzw. einem Säureadditions-Salz oder Metallsalz-Komplex eines Hydroxyalkinyl-azolyl-Derivates der Formel (I).

5. Verfahren zur Bekämpfung von Pilzen, dadurch gekennzeichnet, daß man Hydroxyalkinyl-azolyl-Derivate der Formel (I) in Ansprüchen 1 und 3 bzw. deren Säureadditions-Salze oder Metallsalz-Komplexe auf Pilze oder ihren Lebensraum einwirken läßt.

6. Verwendung von Hydroxyalkinyl-azolyl-Derivaten der Formel (I) in Ansprüchen 1 und 3 bzw. von deren Säureadditions-Salzen und Metallsalz-Komplexen als Pflanzenschutzmittel.

7. Verwendung von Hydroxyalkinyl-azolyl-Derivaten der Formel (I) in Ansprüchen 1 und 3 bzw. von deren Säureadditions-Salzen und Metallsalz-Komplexen zur Bekämpfung von Pilzen.

## Claims

1. Hydroxyalkinyl-azolyl derivatives of the general formula

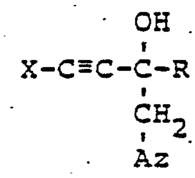

(I)

in which

Az represents 1,2,4-triazol-1-yl or imidazol-1-yl,

X represents straight-chain or branched alkyl with 1 to 4 carbon atoms or phenyl which is optionally mono- to pentasubstituted by identical or different substituents selected from halogen, alkyl with 1 to 4 carbon atoms, alkoxy and alkylthio with in each case 1 to 2 carbon atoms, halogenoalkyl, halogenoalkoxy and halogenoalkylthio with in each case 1 to 2 carbon atoms and 1 to 5 identical or different halogen atoms, the aldoxime radical, the ketoxime radical and ether derivatives thereof, and phenyl, phenoxy, benzyl and benzyloxy, each of which is optionally substituted by halogen and alkyl with 1 to 2 carbon atoms,

R represents the groupings

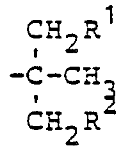 , 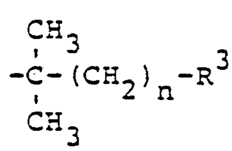 and 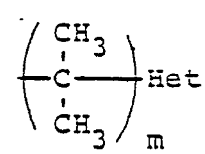

wherein

$R^1$ represents hydrogen, fluorine, chlorine or bromine,

$R^2$ represents fluorine, chlorine or bromine,

$R^3$ represents alkylthio with 1 to 4 carbon atoms, halogenoalkoxy and halogenoalkylthio with in each case 1 to 2 carbon atoms and 1 to 5 identical or different halogen atoms, alkenyl with 2 to 6 carbon atoms, alkoxycarbonyl with 1 to 4 carbon atoms in the alkyl part, cyano and phenyl, phenoxy, phenylthio, phenylalkoxy with 1 to 4 carbon atoms in the alkyl part and phenylalkylthio with 1 to 4 carbon atoms in the alkyl part, each of which is optionally mono- to pentasubstituted by identical or different substituents, the phenyl substituents which may be mentioned in each case being : halogen, alkyl with 1 to 4 carbon atoms ; alkoxy and alkylthio with in each case 1 to 2 carbon atoms ; halogenoalkyl, halogenoalkoxy and halogenoalkylthio with in each case 1 to 2 carbon atoms and 1 to 5 identical or different halogen atoms, cyclohexyl, dialkylamino with 1 to 4 carbon atoms in each alkyl part, nitro, cyano, alkoxycarbonyl with 1 to 4 carbon atoms in the alkyl part, and optionally halogen-substituted phenyl ;

n represents the numbers 0, 1 or 2,

Het represents dioxolan-2-yl or 1,3-dioxanyl, each of which is optionally mono- to tetrasubstituted by identical or different substituents, the substituents which may be mentioned being : alkyl with 1 to 4 carbon atoms and phenyl and phenoxyalkyl with 1 to 4 carbon atoms in the alkyl part, each of which is optionally mono- to pentasubstituted by identical or different substituents, the phenyl substituents which may be mentioned being : halogen, alkyl with 1 to 4 carbon atoms, alkoxy and alkylthio with in each case 1 to 2 carbon atoms and halogenoalkyl, halogenoalkoxy and halogenoalkylthio with in each case 1 to 2 carbon atoms and 1 to 5 identical or different halogen atoms, and

m represents the numbers 0 or 1,

and acid addition salts and metal salt complexes thereof.

2. Hydroxyalkinyl-azolyl derivatives of the formula (I) in Claim 1, wherein

Az represents 1,2,4-triazol-1-yl or imidazol-1-yl,

X represents methyl, ethyl, tert.-butyl and phenyl which is optionally mono- to tri-substituted by identical or different substituents, the substituents which may be mentioned being : fluorine, chlorine, bromine, methyl, tert.-butyl, methoxy, methylthio, trifluoromethyl, trifluoromethoxy, trifluoromethylthio, hydroximinomethyl, 1-hydroximinoethyl, methoximinomethyl, 1-methoximinoethyl and phenyl, phenoxy, benzyl or benzyloxy, each of which is optionally substituted by fluorine, chlorine and methyl,

R represents the groupings

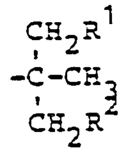 , 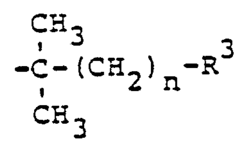 and 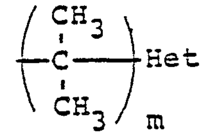

wherein

$R^1$ represents hydrogen, fluorine or chlorine,

$R^2$ represents fluorine or chlorine,

$R^3$ represents methylthio, ethylthio, trifluoromethoxy, trifluoromethylthio, vinyl, methoxycarbonyl, ethoxycarbonyl, cyano and phenyl, phenoxy, phenylthio, phenylmethoxy or phenylmethylthio, each of which is optionally mono- to disubstituted by identical or different substituents, the phenyl substituents which may be mentioned in each case being : fluorine, chlorine, methyl, ethyl, methoxy, methylthio, trifluoromethyl, trifluoromethoxy, trifluoromethylthio, dimethylamino, methoxycarbonyl and ethoxycarbonyl,

n represents the numbers 0, 1 or 2,

Het represents dioxolan-2-yl, 1,3-dioxan-5-yl or 1,3-dioxan-2-yl, each of which is optionally mono- to trisubstituted by identical or different substituents, the substituents which may be mentioned being : methyl, ethyl, n-propyl, isopropyl and phenyl and phenoxymethyl, each of which is optionally mono- to trisubstituted by identical or different substituents selected from fluorine, chlorine, methyl, trifluoromethyl or trifluoromethoxy, and

m represents the numbers 0 or 1.

3. Process for the preparation of hydroxyalkinyl-azolyl derivatives of the general formula

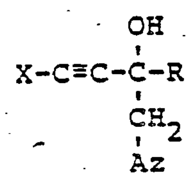 (I)

in which

Az represents 1,2,4-triazol-1-yl or imidazol-1-yl,

X represents straight-chain or branched alkyl with 1 to 4 carbon atoms or phenyl which is optionally mono- to pentasubstituted by identical or different substituents selected from halogen, alkyl with 1 to 4 carbon atoms, alkoxy and alkylthio with in each case 1 to 2 carbon atoms, halogenoalkyl, halogenoalkoxy and halogenoalkylthio with in each case 1 to 2 carbon atoms and 1 to 5 identical or different halogen atoms, the aldoxime radical, the ketoxime radical and ether derivatives thereof, and phenyl, phenoxy, benzyl and benzyloxy, each of which is optionally substituted by halogen and alkyl with 1 to 2 carbon atoms,

R represents the groupings

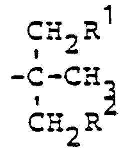 , 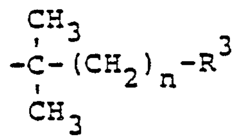 and 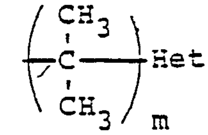

wherein

$R^1$ represents hydrogen, fluorine, chlorine or bromine,

$R^2$ represents fluorine, chlorine or bromine,

$R^3$ represents alkylthio with 1 to 4 carbon atoms, halogenoalkoxy and halogenoalkylthio with in each case 1 to 2 carbon atoms and 1 to 5 identical or different halogen atoms, alkenyl with 2 to 6 carbon atoms, alkoxy-carbonyl with 1 to 4 carbon atoms in the alkyl part, cyano and phenyl, phenoxy, phenylthio, phenylalkoxy with 1 to 4 carbon atoms in the alkyl part and phenyl-alkylthio with 1 to 4 carbon atoms in the alkyl part, each of which is optionally mono- to pentasubstituted by identical or different substituents, the phenyl substituents which may be mentioned in each case being : halogen, alkyl with 1 to 4 carbon atoms ; alkoxy and alkylthio with in each case 1 to 2 carbon atoms ; halogenoalkyl, halogenoalkoxy and halogenoalkythio with in each case 1 to 2 carbon atoms and 1 to 5 identical or different halogen atoms, cyclohexyl, dialkylamino with 1 to 4 carbon atoms in each alkyl part, nitro, cyano, alkoxycarbonyl with 1 to 4 carbon atoms in the alkyl part, and optionally halogen-substituted phenyl ;

n represents the numbers 0, 1 or 2,

Het reprersents dioxolan-2-yl or 1,3-dioxanyl, each of which is optionally mono- to tetrasubstituted by identical or different substituents, the substituents which may be mentioned being : alkyl with 1 to 4 carbon atoms and phenyl and phenoxyalkyl with 1 to 4 carbon atoms in the alkyl part, each of which is optionally mono- to pentasubstituted by identical or different substituents, the phenyl substituents which may be mentioned being : halogen, alkyl with 1 to 4 carbon atoms, alkoxy and alkylthio with in each case 1 to 2 carbon atoms and halogenoalkyl, halogenoalkoxy and halogenoalkylthio with in each case 1 to 2 carbon atoms and 1 to 5 identical or different halogen atoms, and

m represents the numbers 0 or 1,

and acid addition salts and metal salt complexes thereof, characterised in that azolyl-ketones of the general formula (II)

$$Ax—CH_2—CO—R \qquad (II)$$

in which Az and R have the abovementioned meaning, are reacted with acetylene derivatives of the general formula (III)

$$X—C\equiv C—H \qquad (III)$$

in which X has the abovementioned meaning,

in the presence of base and a diluent and, if appropriate, in the presence of a phase transfer catalyst, and, if desired, an acid or a metal salt is added on to the compounds of the formula (I) obtained.

4. Plant protection agents, characterised in that they contain at least one hydroxyalkinyl-azolyl derivative of the formula (I) in Claims 1 and 3 or at least one acid addition salt or metal salt complex of a hydroxyalkinyl-azolyl derivative of the formula (I).

5. Method of combating fungi, characterised in that hydroxyalkinyl-azolyl derivatives of the formula (I) in Claims 1 and 3 or acid addition salts and metal salt complexes thereof are allowed to act on fungi or their environment.

6. Use of hydroxyalkinyl-azolyl derivatives of the formula (I) in Claims 1 and 3 or acid addition salts and metal salt complexes thereof as plant protection agents.

7. Use of hydroxyalkinyl-azolyl derivatives of the formula (I) in Claims 1 and 3 or acid addition salts and metal salt complexes thereof for combating fungi.

**Revendications**

1. Dérivés hydroxyalcinyl-azolyliques de formule générale :

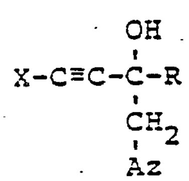

(I)

dans laquelle

Az représente 1,2,4-triazol-1-yle ou imidazol-1-yle,

X un alcoyle à chaîne droite ou ramifiée ayant 1 à 4 atomes de carbone ou un phényle substitué éventuellement une à cinq fois, de manière identique ou différente, par de l'halogène, un alcoyle ayant 1 à 4 atomes de carbone, alcoxy et alcoylthio ayant chacun 1 à 2 atomes de carbone, halogénoalcoyle, halogénoalcoxy et halogénoalcoylthio ayant chacun 1 à 2 atomes de carbone et 1 à 5 atomes d'halogène identiques ou différents, le radical aldoxime, le radical cétoxime et leurs dérivés éther, de même qu'un

phényle, phénoxy, benzyle et benzyloxy éventuellement substitués chacun par de l'halogène et un alcoyle ayant 1 à 2 atomes de carbone,

R les groupements

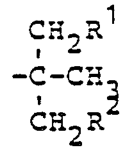 , 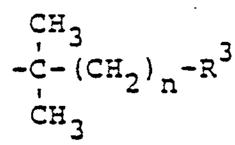 et 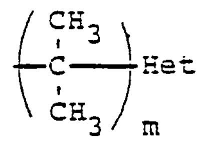

où

$R^1$ représente de l'hydrogène, du fluor, chlore ou brome,

$R^2$ du fluor, chlore ou brome,

$R^3$ un alcoylthio ayant 1 à 4 atomes de carbone, un halogénoalcoxy et halogénotalcoylthio ayant chacun 1 à 2 atomes de carbone et 1 à 5 atomes d'halogène identiques ou différents, un alcényle ayant 2 à 6 atomes de carbone, un alcoxycarbonyle ayant 1 à 4 atomes de carbone dans la portion alcoyle, un cyano de même qu'un phényle, phénoxy, phénylthio, phénylalcoxy substitués chacun une à cinq fois, de manière identique ou différente et avec 1 à 4 atomes de carbone dans la portion alcoyle, et un phénylalcoylthio ayant 1 à 4 atomes de carbone dans la portion alcoyle, en citant chaque fois comme substituants du phényle : halogène, alcoyle ayant 1 à 4 atomes de carbone, alcoxy et alcoylthio ayant chacun 1 à 2 atomes de carbone, halogénoalcoyle, halogénoalcoxy et halogénoalcoylthio ayant chacun 1 à 2 atomes de carbone et 1 à 5 atomes d'halogène identiques ou différents, cyclohexyle, dialcoylamino ayant 1 à 4 atomes de carbone dans chaque portion alcoyle, nitro, cyano, alcoxycarbonyle ayant 1 à 4 atomes de carbone dans la portion alcoyle, de même que phényle éventuellement substitué par de l'halogène,

n représente les nombres 0, 1 et 2,

Het représente un dioxolane-2-yle ou 1,3-dioxanyle substitué éventuellement une à quatre fois, de manière identique ou différente, en citant en tant que substituants : alcoyle ayant 1 à 4 atomes de carbone de même qu'un phényle et phénoxyalcoyle substitués éventuellement chacun une à cinq fois, de manière identique ou différente et avec 1 à 4 atomes de carbone dans la portion alcoyle, en citant comme substituant du phényle : de l'halogène, alcoyle ayant 1 à 4 atomes de carbone, alcoxy et alcoylthio ayant chacun 1 à 2 atomes de carbone, de même que halogénoalcoyle, halogénoalcoxy et halogénoalcoythio ayant chacun 1 à 2 atomes de carbone et 1 à 5 atomes d'halogène identiques ou différents, et

m représente les nombres 0 ou 1,

de même que leurs sels d'addition d'acides et complexes de sels métalliques.

2. Dérivés hydroxyalcinyl-azolyliques de formule (I) selon la revendication 1, dans laquelle

Az représente un 1,2,4-triazol-1-yle ou imidazol-1-yle,

X un méthyle, éthyle, t-butyle de même qu'un phényle éventuellement substitué une à trois fois, de manière identique ou différente, en citant en tant que substituants : fluor, chlore, brome, méthyle, t-butyle, méthoxy, méthylthio, trifluorométhyle, trifluorométhoxy, trifluorométhylthio, hydroximinométhyle, 1-hydroximinoéthyle, méthoximinométhyle, 1-méthoximinoéthyle, de même que phényle, phénoxy, benzyle ou benzyloxy éventuellement substitués chacun par du fluor, chlore et méthyle,

R les groupements

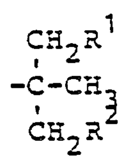 , 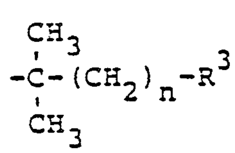 et 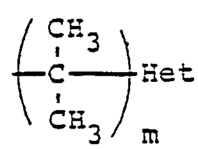

où

$R^1$ représente de l'hydrogène, du fluor ou du chlore,

$R^2$ du fluor ou du chlore,

$R^3$ un méthylthio, éthylthio, trifluorométhoxy, trifluorométhylthio, vinyle, méthoxycarbonyle, éthoxycarbonyle, cyano de même qu'un phényle, phénoxy, phénylthio, phénylméthoxy ou phénylméthylthio éventuellement substitués chacun une à deux fois, de manière identique ou différente, en citant comme substituants du phényle : fluor, chlore, méthyle, éthyle, méthoxy, méthylthio, trifluorométhyle, trifluorométhoxy, trifluorométhylthio, diméthylamino, méthoxycarbonyle et éthoxycarbonyle,

n représente les nombres 0, 1 ou 2,

Het représente un dioxolane-2-yle, 1,3-dioxane-5-yle ou 1,3-dioxane-2-yle éventuellement substitués chacun une à trois fois, de manière identique ou différente, en citant comme substituants : méthyle, éthyle, n-propyle, isopropyle, de même qu'un phényle et phénoxyméthyle éventuellement substitués chacun une à trois fois, de manière identique ou différente par du fluor, chlore, méthyle, trifluorométhyle ou trifluorométhoxy, et

m représente les nombres 0 ou 1.

3. Procédé de préparation de dérivés hydroxyalcinyl-azolyliques de formule générale :

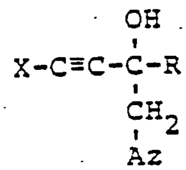 (I)

dans laquelle

Az représente un 1,2,4-triazol-1-yle ou imidazol-1-yle,

X un alcoyle à chaîne droite ou ramifiée ayant 1 à 4 atomes de carbone ou un phényle éventuellement substitué une à cinq fois, de manière identique ou différente, par de l'halogène, un alcoyle ayant 1 à 4 atomes de carbone, alcoxy et alcoylthio ayant chacun 1 à 2 atomes de carbone, halogénoalcoyle, halogénoalcoxy et halogénoalcoylthio ayant chacun 1 à 2 atomes de carbone et 1 à 5 atomes d'halogène identiques ou différents, le radical aldoxime, le radical cétoxime et leurs dérivés éther, de même qu'un phényle, phénoxy, benzyle et benzyloxy éventuellement substitués chacun par de l'halogène et un alcoyle ayant 1 à 2 atomes de carbone,

R les groupements

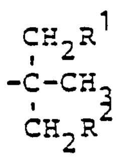 , 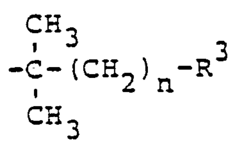 et 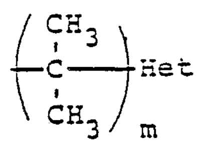 ,

où

$R^1$ représente de l'hydrogène, du fluor, chlore ou brome,

$R^2$ du fluor, chlore ou brome,

$R^3$ un alcoylthio ayant 1 à 4 atomes de carbone, un halogénoalcoxy et halogénoalcoylthio ayant chacun 1 ou 2 atomes de carbone et 1 à 5 atomes d'halogène identiques ou différents, un alcényle ayant 2 à 6 atomes de carbone, un alcoxycarbonyle ayant 1 à 4 atomes de carbone dans la portion alcoyle, un cyano de même qu'un phényle, phénoxy, phénylthio, phénylalcoxy éventuellement substitués chacun une à cinq fois, de manière identique ou différente et ayant 1 à 4 atomes de carbone dans la portion alcoyle et phénylalcoylthio ayant 1 à 4 atomes de carbone dans la portion alcoyle, en citant chaque fois comme substituants du phényle : de l'halogène, alcoyle ayant 1 à 4 atomes de carbone, alcoxy et alcoylthio ayant chacun 1 à 2 atomes de carbone, halogénoalcoyle, halogénoalcoxy et halogénoalcoylthio ayant chacun 1 à 2 atomes de carbone et 1 à 5 atomes d'halogène identiques ou différents, cyclohexyle, dialcoylamino ayant 1 à 4 atomes de carbone dans chaque portion alcoyle, nitro, cyano, alcoxycarbonyle avec 1 à 4 atomes de carbone dans la portion alcoyle, de même que phényle éventuellement substitué par de l'halogène,

n représente les nombres 0, 1 ou 2,

Het représente un dioxolane-2-yle ou 1,3-dioxanyle éventuellement substitué chacun une à quatre fois, de manière identique ou différente, en citant comme substituants : alcoyle ayant 1 à 4 atomes de carbone de même que phényle et phénoxyalcoyle éventuellement substitués chacun une à cinq fois, de manière identique ou différente et ayant 1 à 4 atomes de carbone dans la portion alcoyle, en citant comme substituants du phényle : halogène, alcoyle ayant 1 à 4 atomes de carbone, alcoxy et alcoylthio ayant chacun 1 à 2 atomes de carbone, de même que halogénoalcoyle, halogénoalcoxy et halogénoalcoylthio ayant chacun 1 à 2 atomes de carbone et 1 à 5 atomes d'halogène identiques ou différents, et

m représente les nombres 0 ou 1,

de même que de leurs sels d'addition d'acides et complexes de sels métalliques, caractérisé en ce qu'on fait réagir des azolyl-cétones de formule générale (II)

$$Az—CH_2—CO—R \qquad (II)$$

dans laquelle Az et R ont la signification indiquée plus haut, avec des dérivés d'acétylène de formule générale (III)

$$X—C\equiv C—H \qquad (III)$$

dans laquelle X a la signification indiquée plus haut,

en présence d'une base et d'un diluant de même qu'éventuellement en présence d'un catalyseur de transfert de phase, et en ce qu'éventuellement on fixe sur les composés obtenus de formule (I) un acide ou un sel métallique.

4. Agent de protection des plantes, caractérisé par une teneur en au moins un dérivé hydroxyalcinyl-azolylique de formule (I) selon les revendications 1 et 3, ou bien d'un sel d'addition d'acide ou d'un complexe de sel métallique d'un dérivé hydroxyalcinyl-azolylique de formule (I).

5. Procédé pour combattre les champignons, caractérisé en ce qu'on fait agir des dérivés hydroxyalcinyl-azolyliques de formule (I) selon les revendications 1 et 3, ou bien leurs sels d'addition d'acides ou complexes de sels métalliques, sur les champignons ou sur leur espace vital.

6. Utilisation de dérivés hydroxyalcinylazolyliques de formule (I) selon les revendications 1 et 3, ou bien de leurs sels d'addition d'acides et complexes de sels métalliques, comme agents de protection des plantes.

7. Utilisation de dérivés hydroxyalcinylazolyliques de formule (I) selon les revendications 1 et 3, ou de leurs sels d'addition d'acides et complexes de sels métalliques, pour combattre les champignons.